(19) Europäisches Patentamt
European Patent Office
Office européen des brevets

(11) Veröffentlichungsnummer: **0 189 004**
**A2**

# (12) EUROPÄISCHE PATENTANMELDUNG

(21) Anmeldenummer: **85810609.9**

(22) Anmeldetag: **23.12.85**

(51) Int. Cl.[4]: **C 07 D 401/04**

(30) Priorität: **27.12.84 US 686777**

(43) Veröffentlichungstag der Anmeldung: **30.07.86**
**Patentblatt 86/31**

(84) Benannte Vertragsstaaten: **AT BE CH DE FR GB IT LI LU NL SE**

(71) Anmelder: **CIBA-GEIGY AG, Klybeckstrasse 141, CH-4002 Basel (CH)**

(72) Erfinder: **Boyer, Stephen Kane, Rd 1, Box 204, Far Hills New Jersey 07931 (US)**
Erfinder: **Gelotte, Karl Olaf, 106 Stanie Brae Drive, Watchung New Jersey 07060 (US)**
Erfinder: **Bach, Joseph, 8 Argyle Court, Parsippany New Jersey 07054 (US)**

(54) **Verfahren zur Herstellung bestimmter in 3-Stellung substituierter Indole und Zwischenprodukte dafür.**

(57) Die Erfindung betrifft insbesondere ein Verfahren zur Herstellung der in 3-Stellung substituierten N-Pyridylindole der Formel I

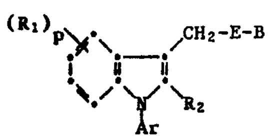

(I)

worin Ar für unsubstituiertes oder durch Niederalkyl substituiertes 3- oder 4-Pyridyl steht, die Reste $R_1$ unabhängig voneinander Wasserstoff, Halogen, Trifluormethyl, Niederalkyl, Hydroxy, acyliertes oder veräthertes Hydroxy oder Niederalkylthio bedeuten, oder zwei an benachbarten Kohlenstoffatomen sitzende Reste $R_1$ Alkylendioxy darstellen, p für 1 oder 2 steht, $R_2$ Wasserstoff oder Niederalkyl bedeutet, E für $C_1$–$C_{11}$-Alkylen, Phenylen, $C_1$–$C_6$-Alkylenphenylen, $C_1$–$C_6$-Alkylen-(thio oder oxy)-niederalkylen, $C_1$–$C_6$-Alkylen-(thio oder oxy)-phenylen, $C_1$–$C_6$-Alkylen-phenylen-niederalkylen, Phenylen-niederalkylen oder eine direkte Bindung steht und B Carboxy, verestertes Carboxy oder Carbamoyl bedeutet, oder ihren pharmazeutisch verwendbaren Salzen, dadurch gekennzeichnet, dass die Ketogruppe in einer Verbindung der Formel IV

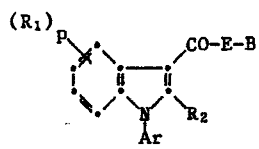

(IV)

worin Ar, $R_1$, $R_2$, p, E und B die unter Formel I gegebenen Bedeutungen haben, selektiv reduziert wird.

**EP 0 189 004 A2**

CIBA-GEIGY AG
Basel (Schweiz)

4-15213/+/CGC 1115

# Verfahren zur Herstellung bestimmter in 3-Stellung substituierter Indole und Zwischenprodukte dafür

Die Erfindung betrifft ein neues, vorteilhaftes Verfahren zur Herstellung bestimmter in 3-Stellung substituierter N-Pyridylindole, die als wertvolle Arzneimittelwirkstoffe bekannt sind. Die Erfindung betrifft ferner neue Ketone, die als Zwischenprodukte zur Herstellung der oben erwähnten pharmakologisch wirksamen Verbindungen nützlich sind und die ebenfalls wertvolle pharmakologische Eigenschaften besitzen, ein Verfahren zur Herstellung dieser Ketone und ihre Verwendung.

Die Erfindung betrifft insbesondere ein Verfahren zur Herstellung der in 3-Stellung substituierten N-Pyridylindole der Formel I

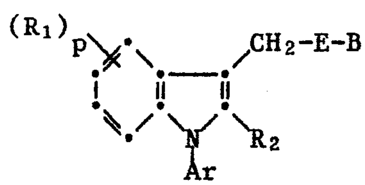

(I)

worin Ar für unsubstituiertes oder durch Niederalkyl substituiertes 3- oder 4-Pyridyl steht, die Reste $R_1$ unabhängig voneinander Wasserstoff, Halogen, Trifluormethyl, Niederalkyl, Hydroxy, acyliertes oder veräthertes Hydroxy oder Niederalkylthio bedeuten, oder zwei an benachbarten Kohlenstoffatomen sitzende Reste $R_1$ Alkylendi-

oxy darstellen, p für 1 oder 2 steht, $R_2$ Wasserstoff oder Niederalkyl bedeutet, E für $C_1$-$C_{11}$-Alkylen, Phenylen, $C_1$-$C_6$-Alkylenphenylen, $C_1$-$C_6$-Alkylen-(thio oder oxy)-niederalkylen, $C_1$-$C_6$-Alkylen-(thio oder oxy)-phenylen, $C_1$-$C_6$-Alkylen-phenylen-niederalkylen, Phenylen-niederalkylen oder eine direkte Bindung steht und B Carboxy, verestertes Carboxy oder Carbamoyl bedeutet, oder ihren pharmazeutisch verwendbaren Salzen, dadurch gekennzeichnet, dass die Ketogruppe in einer Verbindung der Formel IV

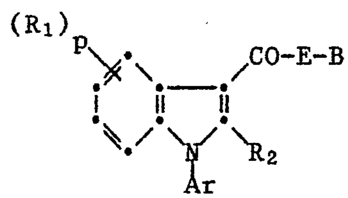

(IV)

worin Ar, $R_1$, $R_2$, p, E und B die unter Formel I gegebenen Bedeutungen haben, selektiv reduziert wird; wobei, falls nötig, störende reaktive Gruppen vorübergehend geschützt sind; und wenn erwünscht, eine erhaltene Verbindung der Formel I in eine andere Verbindung der Formel I umgewandelt wird; und, wenn erwünscht, eine erhaltene freie Verbindung der Formel I in ein Salz oder ein erhaltenes Salz in die freie Verbindung oder in ein anderes Salz überführt wird.

Die Verbindungen der Formel I - einschliesslich der unten angegebenen bevorzugten Verbindungen der Formel II und III - sind als Thromboxan-Synthetase-Hemmer bei Säugern nützlich und sind als solche in der Europäischen Patentanmeldung Nr. 126 401 offenbart. Sie sind daher nützlich zur Behandlung von Krankheiten bei Säugern, die auf eine Hemmung der Thromboxan-Synthetase ansprechen. Solche Krankheiten sind in erster Linie kardiovaskuläre Störungen wie Thrombose, Arteriosklerose, Koronarkrämpfe, celebrale ischämische Anfälle, Migräne und andere vaskuläre Kopfschmerzen, Myocardinfarkt, Angina pectoris und Hypertension.

Alle in EP-A-126 401 angegebenen Verfahren zur Herstellung der Verbindungen der Formel I haben verschiedene Nachteile, z.B. verläuft die Synthese ausgehend von kommerziell erhältlichen Chemikalien über viele Stufen und/oder die Ausbeuten sind niedrig und/oder die benötigten Reagenzien sind teuer. Das dort angegebene Verfahren, das noch relativ am besten zur Herstellung von Verbindungen der Formel I geeignet ist, ist die Kondensation nach Art einer Wittig-Reaktion, z.B. gemäss folgendem Schema:

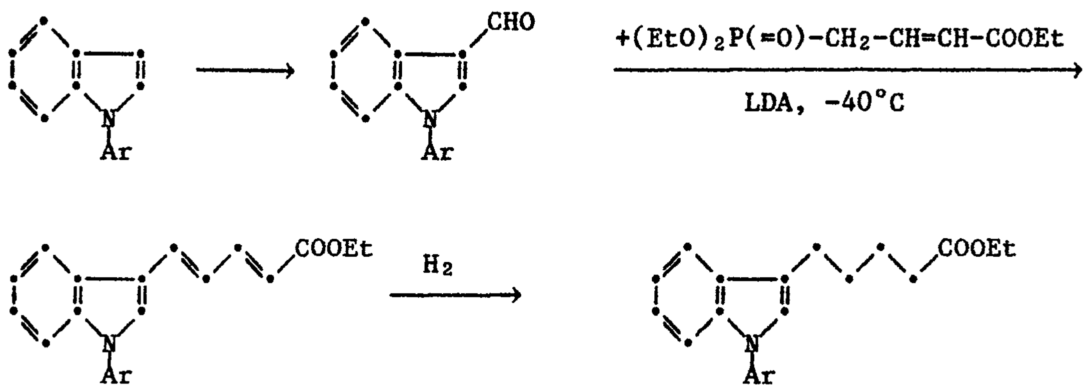


Dieses Verfahren ist gut geeignet zur Durchführung im kleinen Massstab im Labor, seine Anwendung im industriellen Massstab stösst jedoch auf eine Vielzahl von Schwierigkeiten: Die Wittig-artige Reaktion ist im industriellen Bereich sehr schwierig durchzuführen, weil u.a. (a) metallorganische Reagenzien an der Reaktion beteiligt sind, die sehr feuchtigkeitsempfindlich sind und (b) eine Kühlung auf Temperaturen von weit unter 0°C erforderlich ist. Kühlung bei industriellen Verfahren ist bekanntlich extrem teuer, insbesondere dann, wenn wie hier Eiskühlung nicht ausreichend ist.

Das erfindungsgemässe Verfahren erlaubt überraschenderweise die Herstellung der Verbindungen der Formel I im industriellen Massstab, liefert hohe Ausbeuten, erfordert nur wenige Reaktionsstufen ausgehend von kommerziell erhältlichen Chemikalien und ist daher kosteneffektiv. Die gesamte Synthesesequenz verläuft z.B. wie folgt:

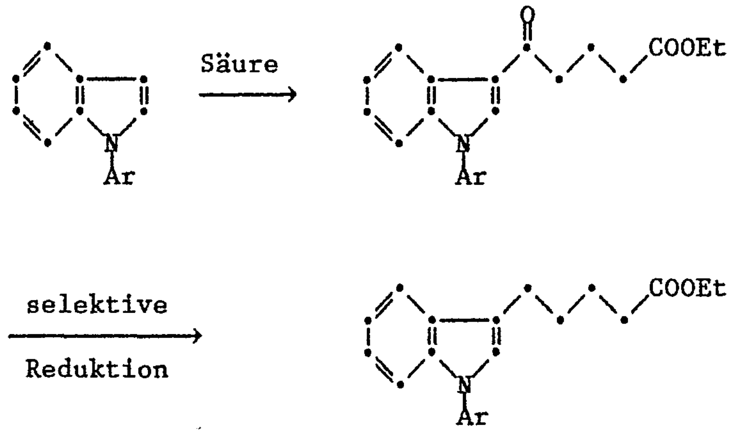


Ausgehend vom gleichen Ausgangsmaterial wie die Wittig-Reaktionssequenz benötigt das neue Verfahren nur zwei (anstelle von drei) Reaktionsstufen, vermeidet die mit dem Einsatz von metallorganischen Reagenzien verbundenen Probleme und macht extrem teure Kühlvorrichtungen unnötig.

Um das erfindungsgemässe Verfahren zu entwickeln, war es nötig, die neuen Ketozwischenprodukte der Formel IV zu konzipieren und eine vorteilhafte Synthese für sie zu entwickeln. Das erfindungsgemässe Verfahren löst überraschenderweise das Problem, eine Ketogruppe in Gegenwart von Carboxy, verestertem Carboxy oder Carbamoyl in hohen Ausbeuten selektiv zu reduzieren.

Die vor-, sowie nachstehend verwendeten Allgemeinbegriffe haben im Rahmen der vorliegenden Erfindung die folgenden Bedeutungen:

$C_1$-$C_{11}$-Alkylen bedeutet Alkylen mit 1 bis 11 Kohlenstoffatomen, das geradkettig oder verzweigt sein kann, und vorzugsweise für Aethylen, Propylen, Butylen, Pentylen, Hexylen oder Heptylen steht, wobei die genannten Reste unsubstituiert oder durch eine oder mehrere Niederalkylgruppen substituiert sind, mit der Massgabe, dass die Summe der Kohlenstoffatome nicht grösser als 11 ist.

Der Ausdruck Phenylen bedeutet 1,2-, 1,3- und vorzugsweise 1,4-Phenylen.

Der Ausdruck "nieder" umfasst in den vor- oder nachstehend genannten organischen Gruppen, Resten oder Verbindungen insbesondere solche mit höchstens 7, vorzugsweise 4 und vor allem 1, 2 oder 3 Kohlenstoffatomen.

$C_1$-$C_6$-Alkylen-phenylen, $C_1$-$C_6$-Alkylen-phenylen-niederalkylen und $C_1$-$C_6$-Alkylen-(thio oder oxy)-phenylen enthalten in jedem Alkylenrest vorzugsweise 1 bis 4 und insbesondere 1 oder 2 Kohlenstoffatome. Die Alkylenreste können geradkettig oder verzweigt sein.

$C_1$-$C_6$-Alkylen-(thio oder oxy)-niederalkylen kann geradkettig oder verzweigt sein und insgesamt 2 bis 11, vorzugsweise 2 bis 8, Kohlenstoffatome haben.

Niederalkyl enthält vorzugsweise 1 bis 4 Kohlenstoffatome und steht z.B für Aethyl, Propyl oder Butyl und insbesondere für Methyl.

Niederalkylen enthält vorzugsweise 1 bis 4 Kohlenstoffatome und steht z.B. für Methylen, Aethylen, 1,3-Propylen oder 1,3- oder 1,4-Butylen.

Niederalkylendioxy bedeutet vorzugsweise Aethylendioxy oder Methylendioxy.

Niederalkoxy enthält vorzugsweise 1 bis 4 Kohlenstoffatome und ist z.B. Aethoxy, Propoxy oder insbesondere Methoxy. Niederalkylthio ist vorzugsweise Methylthio.

Niederalkoxycarbonyl enthält vorzugsweise 1 bis 4 Kohlenstoffatome im Alkoxyteil und bedeutet z.B. Methoxycarbonyl, Propoxycarbonyl oder Isopropoxycarbonyl und insbesondere Aethoxycarbonyl.

Halogen ist vorzugsweise Fluor oder Chlor, kann aber auch Brom oder Jod sein.

Aryl, z.B. in einer Arylniederalkoxy-Gruppe, bedeutet vorzugsweise Phenyl, durch Niederalkyl, Halogen oder Niederalkoxy mono- oder disubstituiertes Phenyl oder Pyridyl.

Arylniederalkoxy ist vorzugsweise Benzyloxy.

Acyliertes Hydroxy bedeutet vorzugsweise Niederalkanoyloxy, z.B. Acetyloxy, unsubstituiertes oder im Phenylring durch Niederalkyl, z.B. Methyl, Halogen, z.B. Chlor, oder Niederalkoxy, z.B. Methoxy, substituiertes Benzoyloxy oder Nikotinoyloxy.

Veräthertes Hydroxy bedeutet vorzugsweise Niederalkoxy, z.B. Methoxy, unsubstituiertes oder im Phenylring durch Niederalkyl, z.B. Methyl, Halogen, z.B. Chlor, oder Niederalkoxy, z.B. Methoxy, substituiertes Benzyloxy oder Pyridylmethoxy.

Unter verestertem Carboxy sind vorzugsweise solche Ester zu verstehen, die pharmazeutisch verwendbar sind, z.B. Niederalkoxycarbonyl, (Amino-, Mono- oder Di-niederalkylamino)-substituiertes Niederalkoxycarbonyl, Carboxy-substituiertes Niederalkoxycarbonyl, z.B. α-Carboxy-substituiertes Niederalkoxycarbonyl, Niederalkoxycarbonyl-substituiertes Niederalkoxycarbonyl, z.B. α-Niederalkoxycarbonyl-substituiertes Niederalkoxycarbonyl, Aryl-substituiertes Niederalkoxycarbonyl, z.B. gegebenenfalls substituiertes Benzyloxycarbonyl oder Pyridylmethoxycarbonyl, (Hydroxy-, Niederalkanoyloxyoder Niederalkoxy)-substituiertes Niederalkoxycarbonyl, z.B. Pivaloyloxymethoxycarbonyl, (Hydroxy-, Niederalkanoyloxy- oder Niederalkoxy)-substituiertes Niederalkoxymethoxycarbonyl, Bicycloalkoxycarbonyl-substituiertes Niederalkoxycarbonyl, z.B. Bicyclo-[2,2,1]-heptyloxycarbonyl-substituiertes Niederalkoxycarbonyl, insbesondere Bicyclo[2,2,1]-heptyloxycarbonyl-substituiertes Methoxy, z.B. Bornyloxycarbonylmethoxycarbonyl, 3-Phthalidoxycarbonyl, (Niederalkyl-, Niederalkoxy-, Halogen)-substituiertes 3-Phthalidoxycarbonyl, Niederalkoxycarbonyloxy-niederalkoxycarbonyl, z.B. 1-(Methoxy- oder Aethoxycarbonyloxy)-äthoxycarbonyl, Aryloxycarbonyl, z.B. Phenoxycarbonyl oder Phenoxycarbonyl, das vorteilhaft in der ortho-Position durch Carboxy oder Niederalkoxycarbonyl substituiert ist.

Pharmazeutisch verwendbare Salze sind insbesondere Metall- oder Ammoniumsalze der genannten Verbindungen der Formel I, welche eine freie Carboxygruppe besitzen, vor allem Alkalimetall- oder Erdalkalimetallsalze, z.B. Natrium-, Kalium-, Magnesium- oder Calciumsalze; in erster Linie leicht kristallisierende Ammoniumsalze, die abgeleitet sind von Ammoniak oder organischen Aminen, z.B. Mono-, Di- oder Tri-nieder-(alkyl, cycloalkyl oder hydroxyalkyl)-aminen, Niederalkylendiaminen oder (Hydroxy-niederalkyl- oder Aryl-niederalkyl)-niederalkylammonium-Hydroxiden, z.B. Methylamin, Diäthylamin, Triäthylamin, Dicyclohexylamin, Triäthanolamin, Aethylendiamin, Tris-(hydroxymethyl)-aminomethan oder Benzyl-trimethylammoniumhydroxid. Die genannten Verbindungen der Formel I bilden Säureadditionssalze. Diese werden vorzugsweise mit solchen anorganischen oder organischen Säuren, die pharmazeutisch verwendbare Säureadditionssalze ergeben, hergestellt. Solche Säuren sind z.B. starke Mineralsäuren, wie Halogenwasserstoffsäuren, z.B. Chlorwasserstoff- oder Bromwasserstoffsäure, Schwefel-, Phosphor-, Salpeter- oder Perchlorsäure; oder organische Säuren, insbesondere aliphatische oder aromatische Carbon- oder Sulfonsäuren, z.B. Essig-, Propion-, Bernstein-, Glykol-, Milch-, Aepfel-, Wein-, Glucon-, Zitronen-, Malein-, Fumar-, Brenztrauben-, Phenylessig-, Benzoe-, 4-Aminobenzoe-, Anthranil-, 4-Hydroxybenzoe-, Salicyl-, 4-Aminosalicyl-, Pamoe-, Nikotin-, Methansulfon-, Aethansulfon-, Hydroxyäthansulfon-, Benzolsulfon-, p-Toluolsulfon-, Naphthalinsulfon-, Sulfanil- oder Cyclohexylsulfaminsäure; oder andere saure organische Substanzen, wie z.B. die Ascorbinsäure.

Bevorzugt ist das Verfahren zur Herstellung von Verbindungen der Formel I, worin Ar für 3-Pyridyl steht, $R_1$ Wasserstoff, Halogen, Trifluormethyl, Niederalkyl, Niederalkoxy, Niederalkylthio, Hydroxy oder Niederalkanoyloxy bedeutet, p für 1 steht, $R_2$ Wasserstoff oder

Niederalkyl ist, E die oben angegebene Bedeutung hat und B Carboxy, Niederalkoxycarbonyl, oder Carbamoyl bedeutet; oder ihren pharmazeutisch verwendbaren Salzen.

Besonders hervorzuheben ist das Verfahren zur Herstellung von Verbindungen der Formel I, worin E für $C_2$-$C_9$-Alkylen, Phenylen, $C_1$-$C_3$-Alkylenphenylen, $C_1$-$C_3$-Alkylen-thio-phenylen oder $C_1$-$C_3$-Alkylen-oxy-phenylen steht und B Carboxy oder Niederalkoxycarbonyl bedeutet, $R_1$ Wasserstoff, Niederalkyl, Halogen, Trifluormethyl, Hydroxy, Niederalkylthio oder Niederalkoxy ist, p für 1 steht, Ar 3-Pyridyl bedeutet und $R_2$ Wasserstoff oder Niederalkyl ist; oder ihren pharmazeutisch verwendbaren Salzen.

In erster Linie ist das Verfahren zur Herstellung von Verbindungen der Formel I bevorzugt, worin E für Alkylen mit 2 bis 7 Kohlenstoffatomen steht.

Von besonderem Interesse ist das Verfahren zur Herstellung von Verbindungen der Formel II

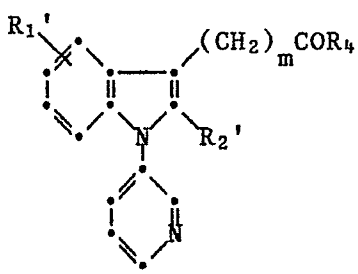

(II)

worin $R_1'$ Wasserstoff, Niederalkyl, Halogen, Trifluormethyl, Hydroxy, Niederalkylthio oder Niederalkoxy bedeutet, $R_2'$ Wasserstoff oder Niederalkyl ist, m für eine ganze Zahl von 1 bis 10 steht und $R_4$ Hydroxy, Niederalkoxy oder Amino bedeutet, oder ihren pharmazeutisch verwendbaren Salzen.

Weiterhin ist das Verfahren zur Herstellung von solchen Verbindungen der Formel II bevorzugt, worin $R_1'$ Wasserstoff, Methyl, Chlor, Fluor, Trifluormethyl, Hydroxy, Methylthio oder Methoxy bedeutet,

$R_2'$ Wasserstoff ist, m für eine ganze Zahl von 3 bis 8 steht und $R_4$ Hydroxy, Aethoxy, Methoxy oder Amino bedeutet; oder ihren pharmazeutisch verwendbaren Salzen.

In allererster Linie ist das Verfahren zur Herstellung von Verbindungen der Formel II bevorzugt, worin $R_1'$ und $R_2'$ Wasserstoff sind, m für 3, 4 oder 5 steht und $R_4$ Hydroxy, Methoxy oder Aethoxy bedeutet; oder ihren pharmazeutisch verwendbaren Salzen.

Bevorzugt ist ferner das Verfahren zur Herstellung von Verbindungen der Formel III

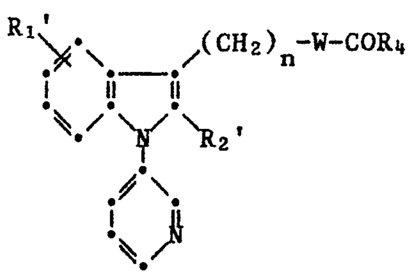

(III)

worin $R_1'$ Wasserstoff, Niederalkyl, Halogen, Trifluormethyl, Hydroxy, Niederalkylthio oder Niederalkoxy bedeutet, $R_2'$ Wasserstoff oder Niederalkyl ist, $R_4$ Hydroxy, Niederalkoxy oder Amino bedeutet; n für eine ganze Zahl von 2 bis 4 steht, aber auch 1 bedeutet, wenn W 1,4-Phenylen ist; und W (Thio oder Oxy)-alkylen mit 1 bis 4 Kohlenstoffatomen, (Thio- oder Oxy)-1,4-phenylen oder 1,4-Phenylen bedeutet; oder ihren pharmazeutisch verwendbaren Salzen.

Vor allen Dingen bevorzugt ist das Verfahren zur Herstellung der in den Beispielen beschriebenen Verbindungen der Formel I (einschliesslich II und III) oder ihren pharmazeutisch verwendbaren Salzen.

Zur Herstellung der bevorzugten Verbindungen der Formel II und III werden die bevorzugten Zwischenprodukte der Formel V bzw. VI verwendet

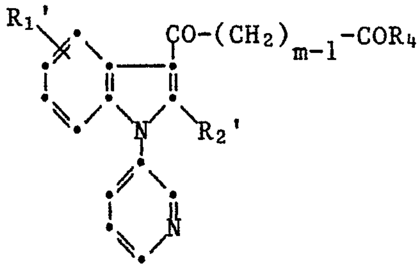

(V)

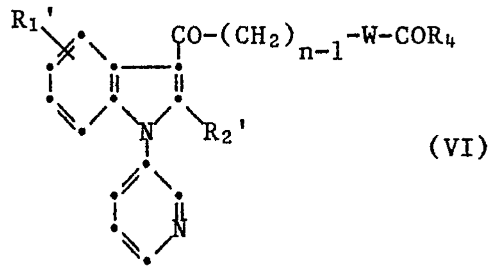

(VI)

worin $R_1'$, $R_2'$, m, n, W und $R_4$ die oben unter Formel II bzw. III angegebene Bedeutung haben.

Gemäss dem erfinderischen Reduktionsverfahren, in dem eine mit dem Indolring direkt verbundene Carbonylgruppe zu Methylen reduziert wird, wird eine Verbindung der Formel IV (einschliesslich V und VI) unter reduktiven Bedingungen, die selektiv für die Reduktion der genannten Carbonylgruppe zu Methylen sind, behandelt, wobei vorteilhaft nur eine geringe oder keine Reduktion anderer funktioneller Gruppen, wie Carboxy, verestertes Carboxy oder Carbamoyl, auftritt.

Ein bevorzugtes Reagenz, um eine solche Reduktion durchzuführen, ist Boran in Form eines Aminkomplexes, z.B. mit einem aliphatischen, aromatischen oder cyclischen Amin, wie tert-Butylamin, Pyridin, N,N-Diäthylanilin, Diisopropylamin, 4-Dimethylaminopyridin, Dimethylamin, 4-Methylmorpholin oder ähnlichen, vorzugsweise unter sauren Bedingungen.

Die Reduktion mit dem Boran-Amin-Komplex wird vorzugsweise in einem sauren polaren Lösungsmittel durchgeführt, vorteilhaft in einer Niederalkansäure, z.B. Essigsäure, mit oder ohne zusätzliches inertes Lösungsmittel, oder in einem inerten Lösungsmittel, z.B. Toluol, Tetrahydrofuran oder Diglyme, in Gegenwart einer Lewissäure, z.B. Aluminiumchlorid oder Zinn(IV)chlorid, bei einer Temperatur von etwa 0°C bis 100°C, vorteilhaft bei oder nahe Raumtemperatur.

Die erfindungsgemässe Reduktion der Carbonylgruppe kann auch durchgeführt werden, indem andere bekannte Verfahren zur Reduktion von Carbonylgruppen angewendet werden, z.B. solche wie sie in

Comprehensive Organic Chemistry Vol. 1, herausgegeben von I.F. Stoddart, Seiten 1079-1081, beschrieben sind. Anwendbar sind z.B. folgende Methoden: Katalytische Hydrierung; Clemmensen-Reduktion, z.B. mit Zinkamalgam oder Zinn unter sauren Bedingungen; Reduktion eines Hydrazonderivates der Carbonylgruppe, z.B. eines Tosylhydrazon-Derivats mit Natriumcyanoborhydrid; reduktive Entschwefelung eines Thioketal-Derivats der Carbonylgruppe, z.B. eines Aethylendithio-Derivates mit Raney-Nickel oder Tributylzinnhydrid in Gegenwart von Azobis-(isobutyronitril).

Eine weitere Methode zur Reduktion der Carbonylgruppe ist die Reduktion mit Diboran.

Die neuen Zwischenprodukte der Formel IV (einschliesslich V und VI) werden hergestellt, indem man unter saurer Katalyse, vorzugsweise in Gegenwart einer Lewis-Säure, eine Verbindung der Formel VII (bzw. VIIa)

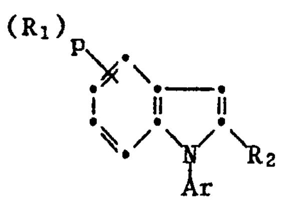 (VII)

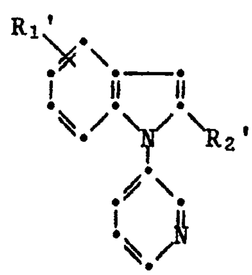 (VIIa)

worin Ar, $R_1$, $R_2$ und p die oben unter Formel I angegebene Bedeutung haben (und $R_1$' und $R_2$' die oben unter Formel II bzw. III angegebene Bedeutung haben), mit einer Verbindung der Formel VIII

HOOC-E-B (VIII)

(oder mit einer Verbindung der Formel VIIIa bzw. VIIIb)

$HOOC-(CH_2)_{m-1}-COR_4$ (VIIIa)

$HOOC-(CH_2)_{n-1}-W-COR_4$ (VIIIb)

worin E und B (m, n, $R_4$ und W) die oben angegebene Bedeutung haben, oder vorzugsweise einem reaktiven funktionellen Derivat davon, acyliert.

Ein reaktives funktionelles Derivat einer Carbonsäure der Formel VIII, VIIIa oder VIIIb ist z.B. ein Acylhalogenid, z.B. das Säurechlorid, ein einfaches Anhydrid im dem Fall, dass B oder $COR_4$ Carboxy bedeuten, ein gemischtes Anhydrid, z.B. eines, dass von einem Kohlensäureniederalkylester-halogenid, wie Chlorameisensäure-äthylester, abgeleitet ist, und ähnliche bekannte Derivate.

Die Kondensation eines Indols der Formel VII oder VIIa mit einer freien Carbonsäure der Formel VIII, VIIIa oder VIIIb wird z.B. in Gegenwart von Polyphosphorsäure oder Polyphosphatester durchgeführt, in einem inerten Lösungsmittel, z.B. Chloroform oder Aethylendichlorid, vorzugsweise bei erhöhter Temperatur.

Die Kondensation eines Indols der Formel VII oder VIIa mit einem reaktiven funktionellen Derivat einer Carbonsäure der Formel VIII, VIIIa oder VIIIb, z.B. einem Säurechlorid davon, wird in Gegenwart einer Lewissäure durchgeführt, z.B. Zinn(IV)chlorid, Titan(IV)chlorid, oder Bortrifluorid-Aetherat, in einem polaren Lösungsmittel, z.B. Methylenchlorid, bei Raumtemperatur oder erhöhter Temperatur, vorteilhaft bei Raumtemperatur.

Die Ausgangsmaterialien der Formel VII und VIIa sind entweder bekannt (z.B. J.Chem. Soc. C 1970, 85) oder werden analog aus den entsprechenden gegebenenfalls substituierten Indolen hergestellt.

Die Ausgangsmaterialien der Formel VIII, VIIIa oder VIIIb und funktionelle Derivate davon sind ebenfalls entweder bekannt oder werden in an sich bekannter Weise hergestellt.

Das oben beschriebene Verfahren liefert überraschenderweise einen vorteilhaften Zugang zu den neuen Zwischenprodukten der Formel IV in hohen Ausbeuten. Die selektive Acylierung von Indolen in 3-Stellung

unter Friedel-Crafts-Bedingungen ist bisher nur in wenigen speziellen Fällen durchgeführt worden, in denen das Indol bestimmmte Substituenten enthielt (z.B. 2-Amino in Tetrahedron Lett. 1971, 3049). Weiterhin sind N-Aryl- oder N-Alkyl- substituierte Indole bisher niemals so acyliert worden. Vielmehr mussten N-Aryl-3-acyl-indole bisher immer auf anderen Wegen hergestellt werden, hauptsächlich durch Reaktion von p-Benzochinon mit geeigneten Aminen oder Iminen oder durch N-Arylierung von 3-Acylindolen. Daher konnte nicht erwartet werden, dass das oben beschriebene Verfahren einen vorteilhaften Zugang zu den Zwischenprodukten der Formel IV liefern würde.

Durch die Bereitstellung der neuen Zwischenprodukte der Formel IV wird ein Syntheseweg eröffnet, auf dem die wertvollen Verbindungen der Formel I in wirtschaftlich und technologisch vorteilhafter Weise hergestellt werden können. Ferner sind die Zwischenprodukte der Formel IV ebenfalls nützlich als Thromboxan-Synthetase-Hemmer.

Das Zweistufenverfahren zur Herstellung der Verbindungen der Formel I, welches die Herstellung einer Verbindung der Formel IV und ihre Reduktion wie oben beschrieben umfasst, bildet einen weiteren Gegenstand der vorliegenden Erfindung.

Die Verbindungen der Formel I (einschliesslich II und III) und die Zwischenprodukte der Formel IV (einschliesslich V und VI) bilden Säureadditionssalze, ferner auch Metall- oder Ammoniumsalze, wenn B (oder $COR_4$) Carboxy bedeutet. Akzeptable Salze sind vorzugsweise die oben beschriebenen pharmazeutisch anwendbaren Salze.

Die oben genannten Reaktionen werden nach an sich bekannten Methoden, in Gegenwart oder Abwesenheit von Verdünnungsmitteln, vorzugsweise in solchen, die gegenüber den Reagenzien inert sind und diese lösen, Katalysatoren, Kondensations- oder anderen obengenannten Mitteln, und/oder einer inerten Atmosphäre, unter Kühlung, bei

Raumtemperatur oder bei erhöhter Temperatur, vorzugsweise beim Siedepunkt des verwendeten Lösungsmittels, bei normalem oder erhöhtem Druck, durchgeführt.

Die Erfindung betrifft ebenfalls Abänderungen des vorliegenden Verfahrens, wonach ein auf irgendeiner Stufe des Verfahrens erhaltenes Zwischenprodukt als Ausgangsmaterial verwendet wird und die verbleibenden Verfahrensschritte durchgeführt werden, oder das Verfahren auf irgendeiner Stufe abgebrochen wird, oder wonach ein Ausgangsmaterial unter den Reaktionsbedingungen gebildet, oder worin einer oder mehrere der Ausgangsstoffe in Form eines Salzes verwendet wird.

Immer wenn es wünschenswert ist, werden bei der Durchführung der oben beschriebenen Verfahren in einem ersten Schritt möglicherweise störende reaktive funktionelle Gruppen im Molekül in an sich bekannter Weise geschützt.

Daher sind in Ausgangsverbindungen und Zwischenprodukten, die wie oben beschrieben zu den Zielverbindungen umgesetzt werden, funktionelle Gruppen z.B. Carboxy und/oder Hydroxy, gegebenenfalls durch Schutzgruppen geschützt, die in der präparativen organischen Chemie üblich sind. Geschützte Carboxy- und Hydroxygruppen zeichnen sich dadurch aus, dass sie unter milden Bedingungen in freie Carboxy- und Hydroxygruppen umgewandelt werden können, ohne dass das Molekülgerüst zerstört wird oder andere unerwünschte Nebenreaktionen stattfinden.

Schutzgruppen, die diese Bedingungen erfüllen, ihre Einführung und Abspaltung werden z.B. in J.F.W. McOmie, "Protective Groups in Organic Chemistry", Plenum Press, London, New York 1973; T.W. Greene, "Protective Groups in Organic Synthesis", Wiley, New York 1984; und ebenso in Houben-Weyl, "Methoden der Organischen Chemie", Vol. 15/1, Georg Thieme Verlag, Stuttgart 1974, beschrieben.

Im Verfahren der vorliegenden Erfindung werden bevorzugt solche Ausgangsstoffe verwendet, die zu den im vorstehenden als besonders wertvoll beschriebenen Verbindungen führen.

Falls Isomerengemische der oben genannten Verbindungen oder Zwischenprodukte erhalten werden, können diese in die einzelnen Isomeren in an sich bekannter Weise aufgetrennt werden, z.B. durch fraktionierte Destillation, Kristallisation oder Chromatographie.

Racemische Endprodukte und Zwischenprodukte können in die optischen Antipoden aufgetrennt werden, indem z.B. diastereomere Salze davon getrennt werden, z.B. durch fraktionierte Kristallisation von d-oder l-(Tartrat, Dibenzoyltartrat, Mandelat oder Camphersulfonat)-Salzen.

Racemische Endstoffe oder saure racemische Zwischenprodukte können auch dadurch aufgespalten werden, dass man z.B. die d- und l-(α-Methylbenzylamin, Cinchonidin, Cinchonin, Chinin, Chinidin, Ephedrin, Dehydroabietylamin, Brucin oder Strychnin)-Salze von solchen Verbindungen, die eine saure salzbindende Gruppe aufweisen, auftrennt.

Die Verbindungen werden entweder in freier Form erhalten oder in Form entsprechender Salze. In Folge der engen Beziehung zwischen den Verbindungen in freier Form und in Form ihrer Salze sind im vorausgegangenen und nachfolgend unter freien Verbindungen und Salzen sinn- und zweckgemäss gegebenenfalls auch die entsprechenden Salze bzw. freien Verbindungen zu verstehen.

Eine erhaltene freie Verbindung kann in ein entsprechendes Säureadditionssalz vorzugsweise mit einer pharmazeutisch akzeptablen Säure oder mit einem Anionenaustauscher überführt werden. Erhaltene Säureadditionssalze können in die entsprechenden freien Verbindungen z.B. durch Behandlung mit einem molaren Aequivalent einer Base, z.B. einem Metall- oder Ammoniumhydroxid oder einem basischen Salz, wie einem Alkalimetallhydroxid oder -carbonat, oder mit einem Kationenaustauscher umgewandelt werden. Freie Carbonsäuren können durch

Behandlung mit Basen in die entsprechenden Metall- oder Ammoniumsalze überführt werden. Diese oder andere Salze können auch zur Reinigung der erhaltenen Verbindungen verwendet werden. Dabei werden die Salze in reiner Form abgetrennt und daraus die freien Verbindungen freigesetzt.

Die Verbindungen und ihre Salze können auch in Form ihrer Hydrate erhalten werden oder andere, für die Kristallisation verwendete Lösungsmittel einschliessen.

Die Verbindungen der Formel I, die durch das erfindungsgemässe Verfahren hergestellt werden, entsprechen den in EP-A-126 401 beschriebenen Verbindungen. Die Identität wird z.B. durch Vergleich des Schmelzpunktes, des $R_f$-Wertes im Dünnschichtchromatogramm oder der Wanderung bei der HPLC-Chromatographie oder durch spektrale Methoden, wie z.B. die IR- und NMR-Spektroskopie, festgestellt.

Die folgenden Beispiele dienen zur Illustration der Erfindung und sind nicht als Einschränkung ihres Umfanges aufzufassen. Temperaturen werden in Celsiusgraden angegeben. Wenn nicht anders angegeben, wird das Eindampfen unter vermindertem Druck, vorzugsweise zwischen ungefähr 20 und 130 mbar, durchgeführt.

Beispiel 1:

a) Zu einer Mischung aus 10,0 g N-(3-Pyridyl)-indol (J. Chem. Soc. C 1970, 85) and 13,8 g Glutarsäuremonoäthylesterchlorid in 90 ml Methylenchlorid wird innerhalb von 3 h tropfenweise eine Lösung von 20,1 g Zinn(IV)chlorid in 60 ml Methylenchlorid gegeben. Das Reaktionsgemisch wird bei Raumtemperatur über Nacht gerührt. Dann wird es auf 10-15° abgekühlt und mit 150 ml 14 % wässriger Ammoniaklösung versetzt. Die organische Phase wird abgetrennt, mit Wasser gewaschen und über Natriumsulfat getrocknet, wobei man 3-(1-Oxo-4-äthoxycarbonylbutyl)-N-(3-pyridyl)-indol vom Smp. 95-97° erhält.

Analog werden hergestellt:

b) 3-(1-Oxo-2-äthoxycarbonyläthyl)-N-(3-pyridyl)-indol;
c) 3-(1-Oxo-3-äthoxycarbonylpropyl)-N-(3-pyridyl)-indol;
d) 5-Brom-3-(1-oxo-4-äthoxycarbonylbutyl)-N-(3-pyridyl)-indol;
e) 3-(1-Oxo-4-äthoxycarbonylbutyl)-2-methyl-N-(3-pyridyl)-indol;
f) 3-(1-Oxo-4-methoxycarbonylbutyl)-7-methyl-N-(3-pyridyl)-indol;
g) 3-(1-Oxo-4-äthoxycarbonylbutyl)-5-methoxy-N-(3-pyridyl)-indol;
h) 3-(1-Oxo-4-äthoxycarbonylbutyl)-5-chlor-N-(3-pyridyl)-indol;
i) 3-(1-Oxo-4-methoxycarbonylbutyl)-5-methyl-N-(3-pyridyl)-indol;
j) 3-(1-Oxo-5-methoxycarbonylpentyl)-N-(3-pyridyl)-indol;
k) 3-(1-Oxo-4-äthoxycarbonylbutyl)-6-chlor-N-(3-pyridyl)-indol.

Die zur Herstellung der oben genannten Verbindungen nötigen Ausgangsmaterialien, die entsprechend substituierten N-(3-pyridyl)-indole, werden aus den entsprechenden N-unsubstituierten Indolen gemäss J. Chem. Soc. C 1970, 85 hergestellt.

Beispiel 2: Eine Mischung aus 15,0 g 3-(1-oxo-4-äthoxycarbonylbutyl)-N-(3-pyridyl)-indol und 100 ml 0,5 N Natronlauge wird 16 h unter Rückfluss gekocht. Die Lösung wird mit Salzsäure auf einen pH-Wert von ungefähr 6 gebracht und mit Methylenchlorid extrahiert. Der Methylenchlorid-Extrakt wird über Natriumsulfat getrocknet und zur Trockene eingedampft, wobei man 3-(1-Oxo-4-carboxybutyl)-N-(3-pyridyl)-indol vom Smp. 177-178° erhält.

3-(1-Oxo-4-carboxybutyl)-N-(3-pyridyl)-indol kann ferner dadurch hergestellt werden, dass man N-(3-Pyridyl)-indol mit Glutarsäureanhydrid in Gegenwart von Zinn(IV)chlorid umsetzt.

Analog werden die Oxo-carbonsäuren der Formel V (worin $R_4$ Hydroxy bedeutet), die den Estern der Beispiele 1b bis 1k) entsprechen, hergestellt.

Beispiel 3:

a) Zu einer Lösung von 5 g 3-(1-Oxo-4-äthoxycarbonylbutyl)-N-(3-pyridyl)-indol in 75 ml Eisessig werden 5 g Boran-tert-Butylamin-Komplex gegeben. Das Reaktionsgemisch wird über Nacht bei Raumtemperatur gerührt. Dann wird es eingeengt, man gibt Wasser zum Rückstand und extrahiert das Produkt mit Methylenchlorid. Die organische Phase wird abgetrennt, zunächst mit konzentrierter Natriumcarbonatlösung, dann mit konzentrierter Kochsalzlösung gewaschen, über Natriumsulfat getrocknet und zur Trockene eingedampft, wobei man 3-(4-Aethoxycarbonylbutyl)-N-(3-pyridyl)-indol vom Smp. 56-58° erhält.

b) Eine Suspension von 0,46 g 3-(4-Aethoxycarbonylbutyl)-N-(3-pyridyl)-indol in 85 ml 1 N Natronlauge wird 8 h auf 95° erhitzt. Die Lösung wird mit Salzsäure neutralisiert und auf einen pH-Wert von etwa 5,5 gebracht. Der entstandene Niederschlag wird abfiltriert und mit Acetonitril verrieben, wobei man 3-(4-Carboxybutyl)-N-(3-pyridyl)-indol (EP-A-126 401) vom Smp. 122-124° erhält.

Beispiel 4: Zu einer Lösung von 0,5 g 3-(1-Oxo-4-äthoxycarbonylbutyl)-N-(3-pyridyl)-indol in 25 ml Tetrahydrofuran werden 0,6 g Boran-tert-Butylamin-Komplex und 0,6 ml Eisessig gegeben. Das Reaktionsgemisch wird auf 90° erhitzt und der Fortgang der Reaktion durch HPLC-Chromatographie verfolgt. Nach vollständiger Reaktion wird das Reaktionsgemisch unter vermindertem Druck eingedampft; Wasser und Methylenchlorid werden hinzugegeben. Der pH-Wert wird durch Zugabe von Natriumcarbonat-Lösung auf 7-8 eingestellt. Die organische Phase wird abgetrennt, mit Wasser gewaschen, getrocknet und zur Trockene eingedampft, wobei man 3-(4-Aethoxycarbonylbutyl)-N-(3-pyridyl)-indol erhält.

Beispiel 5: 3,1 g Mossy-Zink werden mit einem Gemisch aus 0,31 g Quecksilber(II)chlorid, 0,15 ml konzentrierter Salzsäure und 4 ml Wasser 5 min lang behandelt. Dann wird die Lösung dekantiert, 7,5 ml Wasser und 17,5 ml konzentrierte Salzsäure und 10 ml Toluol zugegeben und schliesslich 1 g 3-(1-Oxo-4-äthoxycarbonylbutyl)-N-(3-pyridyl)-indol. Das Reaktionsgemisch wird 24 h unter Rückfluss gekocht. Dann wird es mit 1 N Natronlauge auf einen pH-Wert von 6 eingestellt und mit Methylenchlorid extrahiert. Die Methylenchloridlösung wird mit Wasser gewaschen, über Natriumsulfat getrocknet und zur Trockene eingedampft, wobei man 3-(4-Carboxybutyl)-N-(3-pyridyl)-indol erhält.

Beispiel 6: Zu einer Lösung von 0,5 g 3-(1-Oxo-4-carboxybutyl)-N-(3-pyridyl)-indol in 7,5 ml Eisessig werden 0,5 g Boran-tert-Butylamin-Komplex gegeben. Das Reaktionsgemisch wird bei Raumtemperatur über Nacht gerührt. Es wird eingeengt, der Rückstand in 10 ml 1 N Natronlauge gelöst, Wasser zum Rückstand gegeben und der pH-Wert mit Salzsäure auf 5,0 eingestellt. Der Niederschlag wird abfiltriert und getrocknet, wobei man 3-(4-Carboxybutyl)-N-(3-pyridyl)-indol erhält.

Beispiel 7: Zu einer Lösung von 0,5 g 3-(1-Oxo-4-äthoxycarbonylbutyl)-N-(3-pyridyl)-indol in 15 ml Methylenchlorid werden zunächst 0,40 g Aluminiumchlorid und dann 0,175 g Dimethylamin-Boran-Komplex gegeben. Das Reaktionsgemisch wird 24 h bei Raumtemperatur gerührt. Es wird auf 0°C abgekühlt und tropfenweise mit 10 ml Wasser versetzt. Nach 10 min werden 35 ml 1 N Natronlauge hinzugegeben, um den pH-Wert auf 8 einzustellen. Die Methylenchlorid-Phase wird abgetrennt und die wässrige Phase nochmals mit Methylenchlorid extrahiert. Die vereinigten Methylenchloridextrakte werden mit Wasser gewaschen, getrocknet und zur Trockene eingedampft, wobei man 3-(4-Aethoxycarbonylbutyl)-N-(3-pyridyl)-indol erhält. Die Hydrolyse davon mit verdünnter Natronlauge ergibt wie oben beschrieben 3-(4-Carboxybutyl)-N-(3-pyridyl)-indol.

Beispiel 8: Durch Reduktionsmethoden, die analog zu denen aus den vorhergehenden Beispielen sind, werden die folgenden Verbindungen der Formel II worin $R_4$ Hydroxy oder Aethoxy bedeutet, hergestellt:

| Verbindung | $R_1'$ | $R_2'$ | m |
|---|---|---|---|
| 1 | H | H | 2 |
| 2 | H | H | 3 |
| 3 | 5-Brom | H | 4 |
| 4 | H | $CH_3$ | 4 |
| 5 | 7-Methyl | H | 4 |
| 6 | 5-Methoxy | H | 4 |
| 7 | 5-Chlor | H | 4 |
| 8 | 5-Methyl | H | 4 |
| 9 | H | H | 5 |
| 10 | 6-Chlor | H | 4 |

Patentansprüche für die Vertragsstaaten: BE, CH, DE, FR, GB, IT, LI, LU, NL, SE

1. Verfahren zur Herstellung der in 3-Stellung substituierten N-Pyridylindole der Formel I

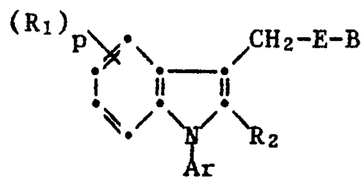

(I)

worin Ar für unsubstituiertes oder durch Niederalkyl substituiertes 3- oder 4-Pyridyl steht, die Reste $R_1$ unabhängig voneinander Wasserstoff, Halogen, Trifluormethyl, Niederalkyl, Hydroxy, acyliertes oder veräthertes Hydroxy oder Niederalkylthio bedeuten, oder zwei an benachbarten Kohlenstoffatomen sitzende Reste $R_1$ Alkylendioxy darstellen, p für 1 oder 2 steht, $R_2$ Wasserstoff oder Niederalkyl bedeutet, E für $C_1$-$C_{11}$-Alkylen, Phenylen, $C_1$-$C_6$-Alkylenphenylen, $C_1$-$C_6$-Alkylen-(thio oder oxy)-niederalkylen, $C_1$-$C_6$-Alkylen-(thio oder oxy)-phenylen, $C_1$-$C_6$-Alkylen-phenylen-niederalkylen, Phenylen-niederalkylen oder eine direkte Bindung steht und B Carboxy, verestertes Carboxy oder Carbamoyl bedeutet, oder ihren pharmazeutisch verwendbaren Salzen, dadurch gekennzeichnet, dass die Ketogruppe in einer Verbindung der Formel IV

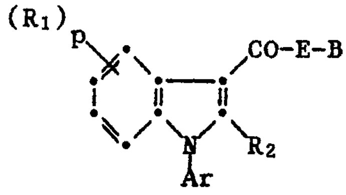

(IV)

worin Ar, $R_1$, $R_2$, p, E und B die unter Formel I gegebenen Bedeutungen haben, selektiv reduziert wird; wobei, falls nötig, störende reaktive Gruppen vorübergehend geschützt sind; und wenn erwünscht, eine erhaltene Verbindung der Formel I in eine andere Verbindung der Formel I umgewandelt wird; und, wenn erwünscht, eine erhaltene freie Verbindung der Formel I in ein Salz oder ein erhaltenes Salz in die freie Verbindung oder in ein anderes Salz überführt wird.

2. Verfahren gemäss Anspruch 1, dadurch gekennzeichnet, dass Verbindungen der Formel I, worin Ar für 3-Pyridyl steht, $R_1$ Wasserstoff, Halogen, Trifluormethyl, Niederalkyl, Niederalkoxy, Niederalkylthio, Hydroxy oder Niederalkanoyloxy bedeutet, p für 1 steht, $R_2$ Wasserstoff oder Niederalkyl ist, E die in Anspruch 1 angegebene Bedeutung hat und B Carboxy, Niederalkoxycarbonyl, oder Carbamoyl bedeutet; oder ihre pharmazeutisch verwendbaren Salze hergestellt werden.

3. Verfahren gemäss Anspruch 1, dadurch gekennzeichnet, dass Verbindungen der Formel I, worin E für $C_2$-$C_9$-Alkylen, Phenylen, $C_1$-$C_3$-Alkylenphenylen, $C_1$-$C_3$-Alkylen-thio-phenylen oder $C_1$-$C_3$-Alkylen-oxy-phenylen steht und B Carboxy oder Niederalkoxycarbonyl bedeutet, $R_1$ Wasserstoff, Niederalkyl, Halogen, Trifluormethyl, Hydroxy, Niederalkylthio oder Niederalkoxy ist, p für 1 steht, Ar 3-Pyridyl bedeutet und $R_2$ Wasserstoff oder Niederalkyl ist; oder ihre pharmazeutisch verwendbaren Salze hergestellt werden.

4. Verfahren gemäss Anspruch 3, dadurch gekennzeichnet, dass Verbindungen der Formel I hergestellt werden, worin E für Alkylen mit 2 bis 7 Kohlenstoffatomen steht.

5. Verfahren gemäss Anspruch 1, dadurch gekennzeichnet, dass die selektive Reduktion mit Boran in Form eines Aminkomplexes unter sauren Bedingungen durchgeführt wird.

6. Verfahren gemäss Anspruch 1, dadurch gekennzeichnet, dass die selektive Reduktion durch reduktive Entschwefelung eines Thioketal-Derivats von einem Keton der Formel IV durchgeführt wird.

7. Verfahren gemäss Anspruch 1, dadurch gekennzeichnet, dass die selektive Reduktion mit Diboran durchgeführt wird.

8. Verfahren gemäss Anspruch 1 zur Herstellung von Verbindungen der Formel II

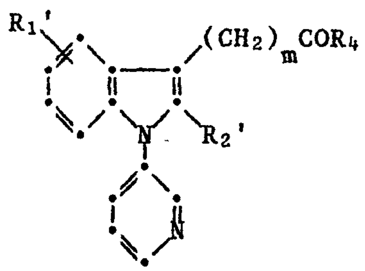

(II)

worin $R_1'$ Wasserstoff, Niederalkyl, Halogen, Trifluormethyl, Hydroxy, Niederalkylthio oder Niederalkoxy bedeutet, $R_2'$ Wasserstoff oder Niederalkyl ist, m für eine ganze Zahl von 1 bis 10 steht und $R_4$ Hydroxy, Niederalkoxy oder Amino bedeutet, oder ihren pharmazeutisch verwendbaren Salzen, dadurch gekennzeichnet, dass die Ketogruppe in einer Verbindung der Formel V

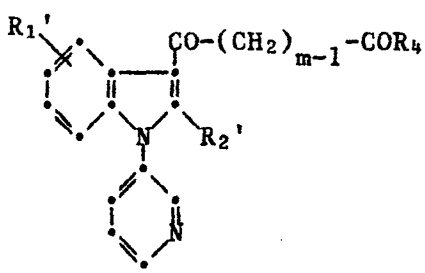

(V)

worin $R_1'$, $R_2'$, m und $R_4$ die unter Formel II angegebene Bedeutung haben, mit Boran in Form eines Aminkomplexes unter sauren Bedingungen selektiv reduziert wird.

9. Verfahren gemäss Anspruch 8, dadurch gekennzeichnet, dass Verbindungen der Formel II, worin $R_1'$ Wasserstoff, Methyl, Chlor, Fluor, Trifluormethyl, Hydroxy, Methylthio oder Methoxy bedeutet, $R_2'$ Wasserstoff ist, m für eine ganze Zahl von 3 bis 8 steht und $R_4$ Hydroxy, Aethoxy, Methoxy oder Amino bedeutet; oder ihre pharmazeutisch verwendbaren Salze hergestellt werden.

10. Verfahren gemäss Anspruch 8, dadurch gekennzeichnet, dass Verbindungen der Formel II, worin $R_1'$ und $R_2'$ Wasserstoff sind, m für 3, 4 oder 5 steht und $R_4$ Hydroxy, Methoxy oder Aethoxy bedeutet; oder ihre pharmazeutisch verwendbaren Salze hergestellt werden.

11. Verfahren gemäss Anspruch 8 zur Herstellung einer Verbindung der Formel II, worin $R_1'$ und $R_2'$ Wasserstoff bedeuten, m für 3, 4 oder 5 steht und $R_4$ Hydroxy bedeutet, dadurch gekennzeichnet, dass die Ketogruppe in einer Verbindung der Formel V, worin $R_1'$ und $R_2'$ Wasserstoff bedeuten, m für 3, 4 oder 5 steht und $R_4$ Niederalkoxy bedeutet, wie in Anspruch 8 beschrieben selektiv reduziert wird und die erhaltene Verbindung der Formel II, worin $R_4$ Niederalkoxy bedeutet, hydrolysiert wird.

12. Verfahren gemäss Anspruch 1, dadurch gekennzeichnet, dass 3-(4-Aethoxycarbonylbutyl)-N-(3-pyridyl)-indol hergestellt wird.

13. Verfahren gemäss Anspruch 1, dadurch gekennzeichnet, dass 3-(4-Carboxybutyl)-N-(3-pyridyl)-indol hergestellt wird.

14. Verfahren gemäss Anspruch 11, dadurch gekennzeichnet, dass 3-(4-Carboxybutyl)-N-(3-pyridyl)-indol hergestellt wird.

15. Verfahren gemäss Anspruch 1 zur Herstellung von Verbindungen der Formel III

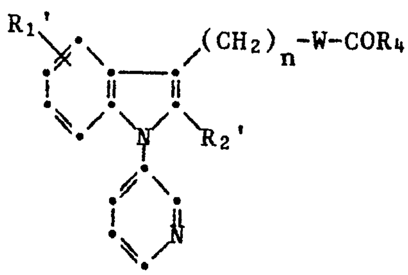

(III)

worin $R_1$' Wasserstoff, Niederalkyl, Halogen, Trifluormethyl, Hydroxy, Niederalkylthio oder Niederalkoxy bedeutet, $R_2$' Wasserstoff oder Niederalkyl ist, $R_4$ Hydroxy, Niederalkoxy oder Amino bedeutet; n für eine ganze Zahl von 2 bis 4 steht, aber auch 1 bedeutet, wenn W 1,4-Phenylen ist; und W (Thio oder Oxy)-alkylen mit 1 bis 4 Kohlenstoffatomen, (Thio- oder Oxy)-1,4-phenylen oder 1,4-Phenylen bedeutet; oder ihren pharmazeutisch verwendbaren Salzen, dadurch gekennzeichnet, dass die Ketogruppe in einer Verbindung der Formel VI

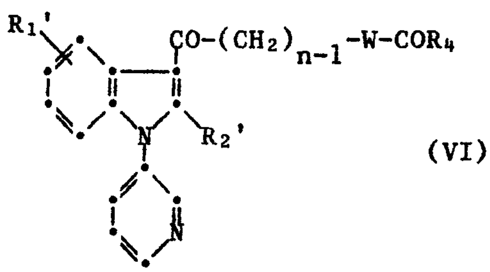

(VI)

worin $R_1$', $R_2$', n, W und $R_4$ die unter Formel III angegebene Bedeutung haben, mit Boran in Form eines Aminkomplexes unter sauren Bedingungen selektiv reduziert wird.

16. Verbindungen der Formel IV

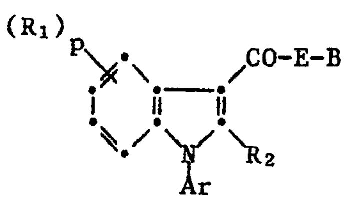

(IV)

worin Ar für unsubstituiertes oder durch Niederalkyl substituiertes 3- oder 4-Pyridyl steht, die Reste $R_1$ unabhängig voneinander Wasserstoff, Halogen, Trifluormethyl, Niederalkyl, Hydroxy, acyliertes oder veräthertes Hydroxy oder Niederalkylthio bedeuten, oder zwei an benachbarten Kohlenstoffatomen sitzende Reste $R_1$ Alkylendioxy darstellen, p für 1 oder 2 steht, $R_2$ Wasserstoff oder Niederalkyl bedeutet, E für $C_1$-$C_{11}$-Alkylen, Phenylen, $C_1$-$C_6$-Alkylenphenylen, $C_1$-$C_6$-Alkylen-(thio oder oxy)-niederalkylen, $C_1$-$C_6$-Alkylen-(thio oder oxy)-phenylen, $C_1$-$C_6$-Alkylen-phenylen-niederalkylen, Phenylen-niederalkylen oder eine direkte Bindung steht und B Carboxy, verestertes Carboxy oder Carbamoyl bedeutet, und pharmazeutisch verwendbare Salze davon.

17. Verbindungen der Formel IV gemäss Anspruch 16, worin E für $C_2$-$C_9$-Alkylen, Phenylen, $C_1$-$C_3$-Alkylenphenylen, $C_1$-$C_3$-Alkylen-thiophenylen oder $C_1$-$C_3$-Alkylen-oxy-phenylen steht und B Carboxy oder Niederalkoxycarbonyl bedeutet, $R_1$ Wasserstoff, Niederalkyl, Halogen, Trifluormethyl, Hydroxy, Niederalkylthio oder Niederalkoxy ist, p für 1 steht, Ar 3-Pyridyl bedeutet und $R_2$ Wasserstoff oder Niederalkyl ist, und pharmazeutisch verwendbare Salze davon.

18. Verbindungen der Formel V gemäss Anspruch 16

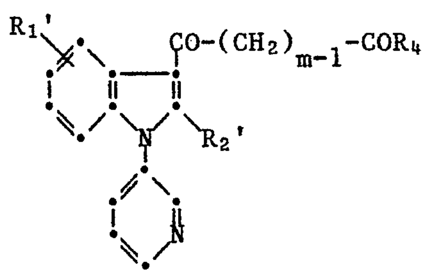

(V)

worin $R_1$' Wasserstoff, Niederalkyl, Halogen, Trifluormethyl, Hydroxy, Niederalkylthio oder Niederalkoxy bedeutet, $R_2$' Wasserstoff oder Niederalkyl ist, m für eine ganze Zahl von 1 bis 10 steht und $R_4$ Hydroxy, Niederalkoxy oder Amino bedeutet, und pharmazeutisch verwendbare Salze davon.

19. Verbindungen der Formel V gemäss Anspruch 18, worin $R_1'$ und $R_2'$ Wasserstoff bedeuten, m für 3, 4 oder 5 steht und $R_4$ Hydroxy, Methoxy oder Aethoxy bedeutet, und pharmazeutisch verwendbare Salze davon.

20. Verbindungen der Formel VI gemäss Anspruch 16

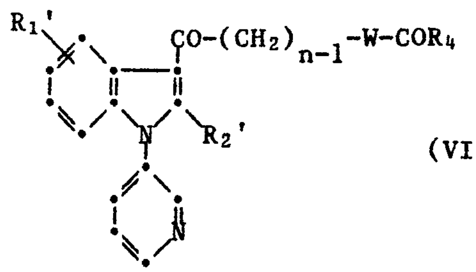

(VI)

worin $R_1'$ Wasserstoff, Niederalkyl, Halogen, Trifluormethyl, Hydroxy, Niederalkylthio oder Niederalkoxy bedeutet, $R_2'$ Wasserstoff oder Niederalkyl ist, $R_4$ Hydroxy, Niederalkoxy oder Amino bedeutet; n für eine ganze Zahl von 2 bis 4 steht, aber auch 1 bedeutet, wenn W 1,4-Phenylen ist; und W (Thio oder Oxy)-alkylen mit 1 bis 4 Kohlenstoffatomen, (Thio- oder Oxy)-1,4-phenylen oder 1,4-Phenylen bedeutet, und pharmazeutisch verwendbare Salze davon.

21. 3-(1-Oxo-4-äthoxycarbonylbutyl)-N-(3-pyridyl)-indol gemäss Anspruch 16 und pharmazeutisch verwendbare Salze davon.

22. 3-(1-Oxo-4-carboxybutyl)-N-(3-pyridyl)-indol gemäss Anspruch 16 und pharmazeutisch verwendbare Salze davon.

23. Verfahren zur Herstellung von Verbindungen der Formel IV gemäss Anspruch 16, dadurch gekennzeichnet, dass man unter saurer Katalyse eine Verbindung der Formel VII

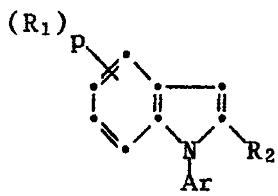

(VII)

worin Ar, $R_1$, $R_2$ und p die unter Formel IV angegebene Bedeutung haben, mit einer Verbindung der Formel VIII

HOOC-E-B (VIII)

worin E und B die unter Formel IV angegebene Bedeutung haben, oder einem reaktiven funktionellen Derivat davon, acyliert; wobei, falls nötig, störende reaktive Gruppen vorübergehend geschützt sind; und wenn erwünscht, eine erhaltene Verbindung der Formel IV in eine andere Verbindung der Formel IV umgewandelt wird; und, wenn erwünscht, eine erhaltene freie Verbindung der Formel IV in ein Salz oder ein erhaltenes Salz in die freie Verbindung oder in ein anderes Salz überführt wird.

Patentansprüche (für den Vertragsstaat: AT)

1. Verfahren zur Herstellung der in 3-Stellung substituierten N-Pyridylindole der Formel I

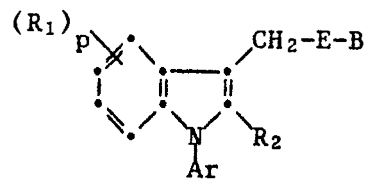

(I)

worin Ar für unsubstituiertes oder durch Niederalkyl substituiertes 3- oder 4-Pyridyl steht, die Reste $R_1$ unabhängig voneinander Wasserstoff, Halogen, Trifluormethyl, Niederalkyl, Hydroxy, acyliertes oder veräthertes Hydroxy oder Niederalkylthio bedeuten, oder zwei an benachbarten Kohlenstoffatomen sitzende Reste $R_1$ Alkylendioxy darstellen, p für 1 oder 2 steht, $R_2$ Wasserstoff oder Niederalkyl bedeutet, E für $C_1$-$C_{11}$-Alkylen, Phenylen, $C_1$-$C_6$-Alkylenphenylen, $C_1$-$C_6$-Alkylen-(thio oder oxy)-niederalkylen, $C_1$-$C_6$-Alkylen-(thio oder oxy)-phenylen, $C_1$-$C_6$-Alkylen-phenylen-niederalkylen, Phenylen-niederalkylen oder eine direkte Bindung steht und B Carboxy, verestertes Carboxy oder Carbamoyl bedeutet, oder ihren pharmazeutisch verwendbaren Salzen, dadurch gekennzeichnet, dass die Ketogruppe in einer Verbindung der Formel IV

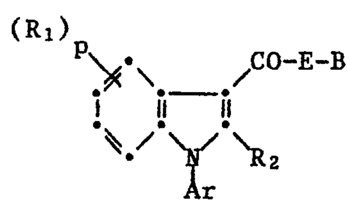

(IV)

worin Ar, $R_1$, $R_2$, p, E und B die unter Formel I gegebenen Bedeutungen haben, selektiv reduziert wird; wobei, falls nötig, störende reaktive Gruppen vorübergehend geschützt sind; und wenn erwünscht, eine erhaltene Verbindung der Formel I in eine andere Verbindung der Formel I umgewandelt wird; und, wenn erwünscht, eine

erhaltene freie Verbindung der Formel I in ein Salz oder ein erhaltenes Salz in die freie Verbindung oder in ein anderes Salz überführt wird.

2. Verfahren gemäss Anspruch 1, dadurch gekennzeichnet, dass Verbindungen der Formel I, worin Ar für 3-Pyridyl steht, $R_1$ Wasserstoff, Halogen, Trifluormethyl, Niederalkyl, Niederalkoxy, Niederalkylthio, Hydroxy oder Niederalkanoyloxy bedeutet, p für 1 steht, $R_2$ Wasserstoff oder Niederalkyl ist, E die in Anspruch 1 angegebene Bedeutung hat und B Carboxy, Niederalkoxycarbonyl, oder Carbamoyl bedeutet; oder ihre pharmazeutisch verwendbaren Salze hergestellt werden.

3. Verfahren gemäss Anspruch 1, dadurch gekennzeichnet, dass Verbindungen der Formel I, worin E für $C_2$-$C_9$-Alkylen, Phenylen, $C_1$-$C_3$-Alkylenphenylen, $C_1$-$C_3$-Alkylen-thio-phenylen oder $C_1$-$C_3$-Alkylen-oxy-phenylen steht und B Carboxy oder Niederalkoxycarbonyl bedeutet, $R_1$ Wasserstoff, Niederalkyl, Halogen, Trifluormethyl, Hydroxy, Niederalkylthio oder Niederalkoxy ist, p für 1 steht, Ar 3-Pyridyl bedeutet und $R_2$ Wasserstoff oder Niederalkyl ist; oder ihre pharmazeutisch verwendbaren Salze hergestellt werden.

4. Verfahren gemäss Anspruch 3, dadurch gekennzeichnet, dass Verbindungen der Formel I hergestellt werden, worin E für Alkylen mit 2 bis 7 Kohlenstoffatomen steht.

5. Verfahren gemäss Anspruch 1, dadurch gekennzeichnet, dass die selektive Reduktion mit Boran in Form eines Aminkomplexes unter sauren Bedingungen durchgeführt wird.

6. Verfahren gemäss Anspruch 1, dadurch gekennzeichnet, dass die selektive Reduktion durch reduktive Entschwefelung eines Thioketal-Derivats von einem Keton der Formel IV durchgeführt wird.

7. Verfahren gemäss Anspruch 1, dadurch gekennzeichnet, dass die selektive Reduktion mit Diboran durchgeführt wird.

8. Verfahren gemäss Anspruch 1 zur Herstellung von Verbindungen der Formel II

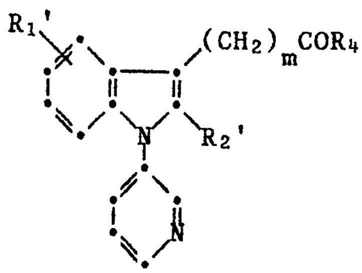

(II)

worin $R_1$' Wasserstoff, Niederalkyl, Halogen, Trifluormethyl, Hydroxy, Niederalkylthio oder Niederalkoxy bedeutet, $R_2$' Wasserstoff oder Niederalkyl ist, m für eine ganze Zahl von 1 bis 10 steht und $R_4$ Hydroxy, Niederalkoxy oder Amino bedeutet, oder ihren pharmazeutisch verwendbaren Salzen, dadurch gekennzeichnet, dass die Ketogruppe in einer Verbindung der Formel V

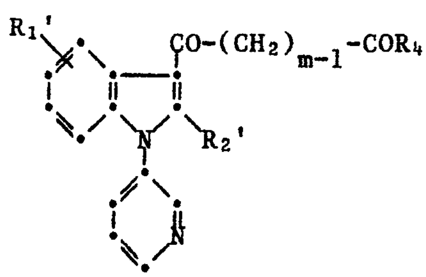

(V)

worin $R_1$', $R_2$', m und $R_4$ die unter Formel II angegebene Bedeutung haben, mit Boran in Form eines Aminkomplexes unter sauren Bedingungen selektiv reduziert wird.

9. Verfahren gemäss Anspruch 8, dadurch gekennzeichnet, dass Verbindungen der Formel II, worin $R_1$' Wasserstoff, Methyl, Chlor, Fluor, Trifluormethyl, Hydroxy, Methylthio oder Methoxy bedeutet, $R_2$' Wasserstoff ist, m für eine ganze Zahl von 3 bis 8 steht und $R_4$ Hydroxy, Aethoxy, Methoxy oder Amino bedeutet; oder ihre pharmazeutisch verwendbaren Salze hergestellt werden.

10. Verfahren gemäss Anspruch 8, dadurch gekennzeichnet, dass Verbindungen der Formel II, worin $R_1'$ und $R_2'$ Wasserstoff sind, m für 3, 4 oder 5 steht und $R_4$ Hydroxy, Methoxy oder Aethoxy bedeutet; oder ihre pharmazeutisch verwendbaren Salze hergestellt werden.

11. Verfahren gemäss Anspruch 8 zur Herstellung einer Verbindung der Formel II, worin $R_1'$ und $R_2'$ Wasserstoff bedeuten, m für 3, 4 oder 5 steht und $R_4$ Hydroxy bedeutet, dadurch gekennzeichnet, dass die Ketogruppe in einer Verbindung der Formel V, worin $R_1'$ und $R_2'$ Wasserstoff bedeuten, m für 3, 4 oder 5 steht und $R_4$ Niederalkoxy bedeutet, wie in Anspruch 8 beschrieben selektiv reduziert wird und die erhaltene Verbindung der Formel II, worin $R_4$ Niederalkoxy bedeutet, hydrolysiert wird.

12. Verfahren gemäss Anspruch 1, dadurch gekennzeichnet, dass 3-(4-Aethoxycarbonylbutyl)-N-(3-pyridyl)-indol hergestellt wird.

13. Verfahren gemäss Anspruch 1, dadurch gekennzeichnet, dass 3-(4-Carboxybutyl)-N-(3-pyridyl)-indol hergestellt wird.

14. Verfahren gemäss Anspruch 11, dadurch gekennzeichnet, dass 3-(4-Carboxybutyl)-N-(3-pyridyl)-indol hergestellt wird.

15. Verfahren gemäss Anspruch 1 zur Herstellung von Verbindungen der Formel III

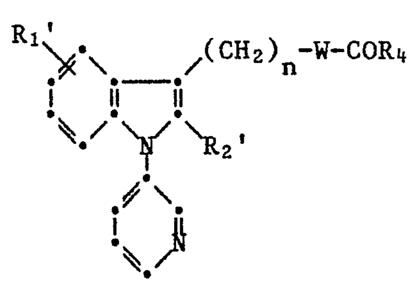

(III)

worin $R_1'$ Wasserstoff, Niederalkyl, Halogen, Trifluormethyl, Hydroxy, Niederalkylthio oder Niederalkoxy bedeutet, $R_2'$ Wasserstoff oder Niederalkyl ist, $R_4$ Hydroxy, Niederalkoxy oder Amino bedeutet; n für eine ganze Zahl von 2 bis 4 steht, aber auch 1 bedeutet, wenn W 1,4-Phenylen ist; und W (Thio oder Oxy)-alkylen mit 1 bis 4 Kohlenstoffatomen, (Thio- oder Oxy)-1,4-phenylen oder 1,4-Phenylen bedeutet; oder ihren pharmazeutisch verwendbaren Salzen, dadurch gekennzeichnet, dass die Ketogruppe in einer Verbindung der Formel VI

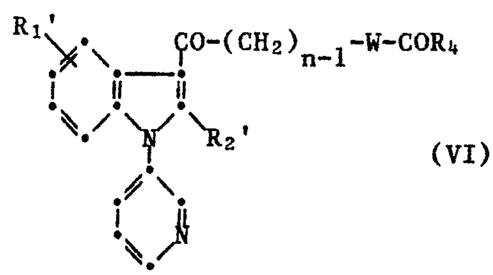

(VI)

worin $R_1'$, $R_2'$, n, W und $R_4$ die unter Formel III angegebene Bedeutung haben, mit Boran in Form eines Aminkomplexes unter sauren Bedingungen selektiv reduziert wird.

16. Verfahren zur Herstellung von Verbindungen der Formel IV

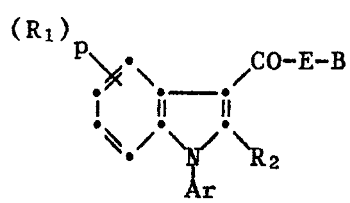

(IV)

worin Ar für unsubstituiertes oder durch Niederalkyl substituiertes 3- oder 4-Pyridyl steht, die Reste $R_1$ unabhängig voneinander Wasserstoff, Halogen, Trifluormethyl, Niederalkyl, Hydroxy, acyliertes oder veräthertes Hydroxy oder Niederalkylthio bedeuten, oder zwei an benachbarten Kohlenstoffatomen sitzende Reste $R_1$ Alkylendioxy darstellen, p für 1 oder 2 steht, $R_2$ Wasserstoff oder Niederalkyl bedeutet, E für $C_1$-$C_{11}$-Alkylen, Phenylen, $C_1$-$C_6$-Alkylenphenylen, $C_1$-$C_6$-Alkylen-(thio oder oxy)-niederalkylen, $C_1$-$C_6$-Alkylen-(thio oder oxy)-phenylen, $C_1$-$C_6$-Alkylen-phenylen-nieder-

alkylen, Phenylen-niederalkylen oder eine direkte Bindung steht und B Carboxy, verestertes Carboxy oder Carbamoyl bedeutet, oder pharmazeutisch verwendbaren Salzen davon, dadurch gekennzeichnet, dass man unter saurer Katalyse eine Verbindung der Formel VII

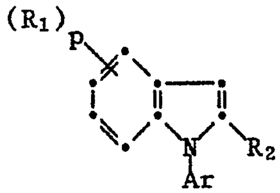

(VII)

worin Ar, $R_1$, $R_2$ und p die unter Formel IV angegebene Bedeutung haben, mit einer Verbindung der Formel VIII

HOOC-E-B (VIII)

worin E und B die unter Formel IV angegebene Bedeutung haben, oder einem reaktiven funktionellen Derivat davon, acyliert; wobei, falls nötig, störende reaktive Gruppen vorübergehend geschützt sind; und wenn erwünscht, eine erhaltene Verbindung der Formel IV in eine andere Verbindung der Formel IV umgewandelt wird; und, wenn erwünscht, eine erhaltene freie Verbindung der Formel IV in ein Salz oder ein erhaltenes Salz in die freie Verbindung oder in ein anderes Salz überführt wird.

17. Verfahren gemäss Anspruch 16 zur Herstellung von Verbindungen der Formel IV, worin E für $C_2$-$C_9$-Alkylen, Phenylen, $C_1$-$C_3$-Alkylen-phenylen, $C_1$-$C_3$-Alkylen-thio-phenylen oder $C_1$-$C_3$-Alkylen-oxy-phenylen steht und B Carboxy oder Niederalkoxycarbonyl bedeutet, $R_1$ Wasserstoff, Niederalkyl, Halogen, Trifluormethyl, Hydroxy, Niederalkylthio oder Niederalkoxy ist, p für 1 steht, Ar 3-Pyridyl bedeutet und $R_2$ Wasserstoff oder Niederalkyl ist, oder pharmazeutisch verwendbaren Salzen davon.

18. Verfahren gemäss Anspruch 16 zur Herstellung von Verbindungen der Formel V

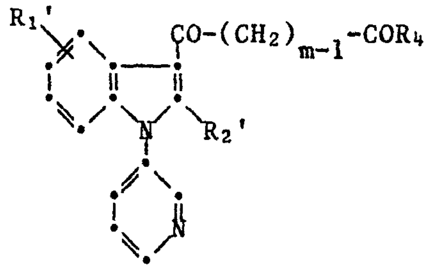

(V)

worin $R_1'$ Wasserstoff, Niederalkyl, Halogen, Trifluormethyl, Hydroxy, Niederalkylthio oder Niederalkoxy bedeutet, $R_2'$ Wasserstoff oder Niederalkyl ist, m für eine ganze Zahl von 1 bis 10 steht und $R_4$ Hydroxy, Niederalkoxy oder Amino bedeutet, oder pharmazeutisch verwendbaren Salzen davon.

19. Verfahren gemäss Anspruch 18 zur Herstellung von Verbindungen der Formel V, worin $R_1'$ und $R_2'$ Wasserstoff bedeuten, m für 3, 4 oder 5 steht und $R_4$ Hydroxy, Methoxy oder Aethoxy bedeutet, oder pharmazeutisch verwendbaren Salzen davon.

20. Verfahren gemäss Anspruch 16 zur Herstellung von Verbindungen der Formel VI

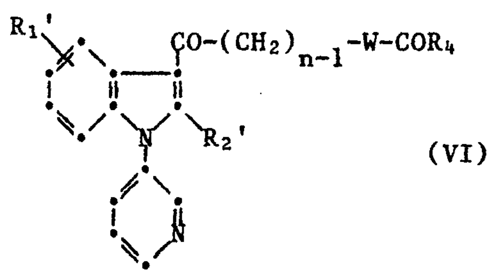

(VI)

worin $R_1'$ Wasserstoff, Niederalkyl, Halogen, Trifluormethyl, Hydroxy, Niederalkylthio oder Niederalkoxy bedeutet, $R_2'$ Wasserstoff oder Niederalkyl ist, $R_4$ Hydroxy, Niederalkoxy oder Amino bedeutet; n für eine ganze Zahl von 2 bis 4 steht, aber auch 1 bedeutet, wenn W 1,4-Phenylen ist; und W (Thio oder Oxy)-alkylen mit 1 bis 4 Kohlenstoffatomen, (Thio- oder Oxy)-1,4-phenylen oder 1,4-Phenylen bedeutet, oder pharmazeutisch verwendbaren Salzen davon.

21. Verfahren gemäss Anspruch 16 zur Herstellung von 3-(1-Oxo-4-äthoxycarbonylbutyl)-N-(3-pyridyl)-indol oder einem pharmazeutisch verwendbaren Salz davon.

22. Verfahren gemäss Anspruch 16 zur Herstellung von 3-(1-Oxo-4-carboxybutyl)-N-(3-pyridyl)-indol oder einem pharmazeutisch verwendbaren Salz davon.

FO 7.4 BL/bg*